(19) Europäisches Patentamt
European Patent Office
Office européen des brevets

(11) **EP 3 326 598 A1**

(12) **EUROPEAN PATENT APPLICATION**

(43) Date of publication:
**30.05.2018 Bulletin 2018/22**

(51) Int Cl.:
*A61F 13/514* (2006.01)
*A61F 13/15* (2006.01)
*B41F 5/24* (2006.01)
*A61F 13/84* (2006.01)
*B41F 5/18* (2006.01)

(21) Application number: **17203266.6**

(22) Date of filing: **23.11.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.11.2016 US 201662426664 P**

(71) Applicant: **The Procter & Gamble Company
Cincinnati, OH 45202 (US)**

(72) Inventors:
• **BLANCO TORRES, Priscila
Cincinnati, OH 45202 (US)**
• **KINDSCHUH, Olivia
Cincinnati, OH 45202 (US)**
• **MALDONADO, Clarissa
Cincinnati, OH 45202 (US)**
• **WARNER, Alrick Vincent
Cincinnati, OH 45202 (US)**

(74) Representative: **Chandrani, Vandita
Procter & Gamble Service GmbH
IP Department
Sulzbacher Strasse 40 - 50
65824 Schwalbach am Taunus (DE)**

(54) **METHOD OF CREATING MULTIPLE PATTERNS**

(57)    A method of continuously imparting different visually detectable features to a plurality of articles is provided. The method includes providing a printing unit having two or more printing rolls each having a visually detectable pattern, wherein the printing process may fit rolls that are less than or equal to a maximum circumference. The method further includes providing a web having a print pitch that is greater than one half the maximum circumference, providing a first roll wherein the roll circumference is equal to the product pitch, and providing a second roll characterized in that the roll circumference is equal to either $0<X<1$ where X is chosen such that $1/X \neq$ an Integer or $1<X< C_{max}/P$, wherein X is a multiple of the first roll circumference, $C_{max}$ is the largest circumference allowable by the printing unit, and P is the product pitch. The method further includes imparting the visually detectable patterns to the web by means of the two or more rolls.

Fig. 1

**Description**

FIELD OF INVENTION

This application relates to a method of continuously imparting visually detectable features to a plurality of items. The disclosure also relates to a package including a plurality of items wherein each item within said package includes a visually detectable feature.

BACKGROUND OF THE INVENTION

Absorbent products intended to absorb discharged body fluids are well known in the art. Such absorbent products generally comprise a fibrous mass or other absorbent body which can absorb and hold body fluids. The absorbent articles have included various systems of liquid-handling layers, such as intake layers, distribution layers, retention layers and the like. To improve aesthetic appeal, the absorbent products may have graphics on either the body side liner, the garment side liner, or on a wrapper.

Selecting a particular design for the personal care articles as well as the wrappers can be a deciding factor. While being liked by some consumers, other consumers may dislike the design. Additionally, many consumers desire multiple designs within one package versus having only one design.

Additionally, the garment contacting surface of the absorbent articles often has printed graphics to get a positive attention from the user or caregiver not only in use but also in the market. In the latter case, the absorbent articles are often contained in a package having a transparent portion such that the graphics are visible through the transparent portion. Such graphics are typically printed on either a landing zone material, a wrapper for the absorbent article, a topsheet, a secondary topsheet, a fastening tape, a reseal tape, nonwoven covers, or a backsheet material. Graphics printed on the garment contacting surface of disposable absorbent articles can be preferred by consumers due to their entertaining functions. Additionally, graphics allows the user to distinguish different parts of the product, e.g. the front versus the back.

Those graphics are typically printed by using conventional printing techniques such as gravure and flexography technologies which employ a printing plate or sleeve to print the graphic on a sheet material, e.g. a backsheet material, a landing zone material, a fastening tape material or a nonwoven material. In the flexographic printing process, Ink is transferred from an ink fountain thru a series of rolls onto the raised areas of the printing plate or sleeve which is mounted onto a cylinder which rotates at a rotational speed equal or close to the speed of the flexible sheet material, coming in contact with and transferring the ink to the sheet creating an image on its surface.

In the gravure process the desired image to be printed is engraved in a metal cylinder creating recessed areas. The metal cylinder is partially immersed in a bath of ink and a blade wipes any excess ink leaving only the recessed areas with ink. The flexible sheet material comes in contact with the metal cylinder which then transfers the ink from the recessed areas to the surface of the sheet material, transferring an image.

Traditionally, a process will print a set pattern. Dependent upon the product pitch the ability to print patterns may be limited. This is because flexographic and gravure printing processes are limited on the sizes and number of images that can be printed, which results only in a very limited number of designs available from a single process. In essence, dependent upon the product pitch and the physical real world constraints of the process, one may only be able to fit a single pitch on the largest roll thereby limiting the number of graphics.

Hence, there is a need for a method that can provide a large number of different patterns created by a plurality of graphics imparted on a web for use in an absorbent articles or a wrapper, using one process.

SUMMARY OF THE INVENTION

Provided is a method of continuously imparting different visually detectable features to a plurality of articles. The method includes providing a printing unit having two or more printing rolls each having a visually detectable pattern, wherein the printing process may fit rolls that are less than or equal to a maximum circumference. Providing a web having a print pitch that is greater than one half the maximum circumference. Providing a first roll wherein the roll circumference is equal to the product pitch. Providing a second roll characterized in that the roll circumference is equal to either $0 < X < 1$ where X is chosen such that $1/X \neq$ an Integer or $1 < X < C_{max}/P$, wherein X is a multiple of the first roll circumference, $C_{max}$ is the largest circumference allowable by the printing unit, and P is the product pitch. The method further includes imparting the visually detectable patterns to the web by means of the two or more rolls.

Also provided is a method of continuously imparting different visually detectable features to a plurality of articles. The method including providing a printing unit comprising of two or more printing rolls each having a visually detectable pattern, wherein the printing process may fit rolls that are less than or equal to a maximum circumference; providing a web comprising a print pitch; providing a first roll wherein the roll circumference is equal to the product pitch; providing a second roll characterized in that the roll circumference is equal to either 0<X<1 where X is chosen such that $1/X \neq$ an Integer or 1<X< $C_{max}$/P, wherein X is a multiple of the first roll circumference, $C_{max}$ is the largest circumference allowable by the printing unit, and P is the product pitch; and imparting the visually detectable patterns to the web by means the one or more rolls. The method is characterized in that each of the first roll and the second roll comprises a visually detectable pattern defining a print length, wherein the print length is characterized in that the absolute of (X-1) / (print length/ product pitch)) is greater than 1.

BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:

FIG. 1 shows a schematic of an embodiment of the method.
FIG. 2 shows an example of the method.
FIG. 3A-C shows a perspective view of absorbent articles according to the example of FIG. 2.
FIG. 4A-C shows a plan view of the absorbent articles of FIG. 3A-C.
Fig. 5 is a plan view of an example diaper, garment-facing surface facing the viewer, in a flat laid-out state.
Fig. 6 is a plan view of the example diaper of Fig. 5, wearer-facing surface facing the viewer, in a flat laid-out state.
Fig. 7 is a front perspective view of the diaper of Figs. 5 and 6 in a fastened position.
Fig. 8 is a front perspective view of a pant.
Fig. 9 is a rear perspective view of the pant of Fig. 8.
Fig. 10 is a plan view of the pant, laid flat, with a garment-facing surface facing the viewer.
Fig. 11 is a cross-sectional view of a front belt portion taken about line 7---7 of Fig. 10.
Fig. 12 is a cross-sectional view of a back belt portion taken about line 8-8 of Fig. 10.
Fig. 13 is a plan view of an example absorbent article of the present disclosure that is a sanitary napkin.

DETAILED DESCRIPTION OF THE INVENTION

The present invention is directed to a method of creating a plurality of visual patterns on a web using two or more rolls contained within one process. At least one of the first or second rolls has a plurality of patterns wherein each pattern relates to a product length. The second roll may have the same consistent pattern or a plurality of patterns. The patterns are designed such that they come together to create a pleasant aesthetic design.
Additionally, the application is directed to a method of creating a plurality of visual patterns on a web using two or more rolls contained within one process wherein the second roll is has a pitch that is a multiple of the first roll pitch.. This allows for the second roll to create a plurality of consistent repeating patterns. For example, a pitch multiple of 1.5 allows one to create three distinct patterns that repeat. The patterns are designed such that they come together to create a pleasant aesthetic design. Additional rolls may be added to the process such that additional graphical elements may be added for every certain number of products. The method will be described in further detail below.
The following text sets forth a broad description of numerous different embodiments of the present invention. The description is to be construed as exemplary only and does not describe every possible embodiment since describing every possible embodiment would be impractical, if not impossible. And it will be understood that any feature, characteristic, component, composition, ingredient, product, step or methodology described herein can be deleted, combined with or substituted for, in whole or part, any other feature, characteristic, component, composition, ingredient, product, step or methodology described herein. Numerous alternative embodiments could be implemented, using either current technology or technology developed after the filing date of this patent, which would still fall within the scope of the claims. All publications and patents cited herein are incorporated herein by reference.
It should also be understood that, unless a term is expressly defined in this specification using the sentence "As used herein, the term '_' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). No term is intended to be essential to the present invention unless so stated. To the extent that any term recited in the claims at the end of this patent is referred to in this patent in a manner consistent with a single meaning, that is done for sake of clarity only so as to not confuse the reader, and it is not intended that such a claim term be limited, by implication or otherwise, to that single meaning. Finally, unless a claim element is defined by reciting the word "means" and a function without the recital of any structure, it is not intended that the scope of any claim element be interpreted based on the application of 35 U.S.C. § 112, sixth paragraph.
As used herein, the term "different" means that the visually detectable features are perceived as different by a user when the user looks at the visually detectable features using his/her usual attention. Thus, two visually detectable features having differences which are unintentionally caused by manufacturing variations or errors are not different from each other. Different visually detectable features are determined by, for example, different components constituting visually detectable features, different colours or colour combinations used in the visually detectable features or its components, different sizes of the visually detectable features or its components, the spatial orientation of the visually detectable features or its components, the geometrical shapes of the visually detectable features or its components, the number of the components in the visually detectable features, or the like.

As used herein, the term "different printed element" is intended to mean that there is at least one aspect of the printed element which is different. A different printed element can include, for example, objects which are not the same size, colors which are not the same, a change in spatial orientation of objects of the printed element, or other aspects which make it apparent to the consumer that the printed elements are different. The printed elements can be printed with any process known to those skilled in the art.

By "item" is meant a single product imparted with a visually detectable feature according to the method. An item in the context of the present description may be an absorbent article such as a diaper, a sanitary napkin, a panty liner, an incontinence protector or the like; a wrapping sheet, such as a single wrap or easy wrap; or a release liner.

As used herein, the term "printed element" includes but is not limited to a color, a geometrical shape, a character, a symbol, a letter, text, a pattern, a design, and combinations thereof.

The disclosed method allows one to create "registered" patterns. As used herein, "registered" patterns represents that the same pattern may be created such that visually detectable elements are in the same location. As disclosed in the specification, dependent upon the dimension of the rolls chosen a repeat cycle can be calculated, the registered patterns will then repeat dependent upon that cycle as exemplified by the examples. For example, a pattern may repeat every sixth pattern. That stated, the first and seventh pattern will be identical thereby being "registered" as defined herein.

By "visually detectable element" is meant a portion of the roll having a pattern, a symbol, an object or a colour to be imparted on a web.

As used herein, the term "visually detectable pattern element" means a pattern, a symbol, an object or a colour transferred by means of the visually detectable element to the item.

By "visually detectable feature" is meant the composition of all the visually detectable pattern elements on one item. It is noteworthy that different visually detectable features may comprise at least portions of the same visually detectable pattern element.

A visually detectable feature may be a printed and/or embossed pattern, a printed and/or embossed image, a colour or a combination thereof.

Disposable absorbent articles such as, for example, feminine care, diapers, and incontinent absorbent products, generally include a liquid pervious topsheet, a substantially liquid impervious backsheet, and an absorbent core positioned and held between the topsheet and the backsheet. Disposable absorbent articles may also include other optional components or layers, such as liquid wicking layers, liquid distribution layers, barrier layers, and the like, as well as combinations thereof, which may improve the fluid handling and storage properties of the disposable absorbent article. Generally, disposable absorbent articles provide a body-facing surface and a garment-facing surface. The body-facing surface is generally the topsheet and garment facing surface is the backsheet. The visually detectable features may be present on any component of the absorbent article. Disposable absorbent articles are discussed in further detail below.

The wrapping sheet is intended to protect the absorbent article from outer factors such as dirt and damage. The wrapping sheet may contain one or several absorbent articles. The material of the wrapping sheet may be any material known in the art, such as polyethylene or propylene film, a nonwoven material, or a laminate of a nonwoven material and a plastic film. The visually detectable feature may be present on the outer and/or the inner side of the wrapping material, or between the layers if several layers are present.

Release liner is a component provided for protection of the adhesive provided on the garment-facing sheet of absorbent article. The release liner can be of any material known in the art, e.g. silicone-coated paper. The visually detectable feature may be present on one or both sides of the release liner.

The web may be used as any part of an absorbent article including the backsheet, the topsheet, or the secondary topsheet. The web may be used as a wrapper for the absorbent article, a package for a plurality of absorbent articles, or a release sheet for an absorbent article. The web may be used as part of a package. The web may be used in any product that utilizes a web with graphical elements. Other uses of the web not contemplated herein are not excluded.

As stated above, the method relates to the use of multiple rolls to create a plurality of patterns for a web to be used in absorbent articles. The method includes providing a flexographic or gravure printing process capable of utilizing multiple rolls. The method further includes setting a first roll circumference to the product pitch. Once the first roll diameter and circumference are known, additional rolls such as, for example, a second roll and a third roll are chosen. The second and third roll should have a pitch driven by the circumference that is a multiple of the first roll.

The method may be used when a product pitch is larger than one half the circumference of the largest possible roll dimension that may fit in the flexographic or gravure printing process. Often, the largest roll diameter may be due to real world process or space limitations. Traditionally, under this circumstance, a process could only produce on fixed pattern. It has been found that by setting a first roll to the product pitch and any additional rolls (second, third, fourth, five....) to a multiple that is greater than zero and less than (the maximum circumference allowed in the system/ product pitch), then one can create a plurality of controlled patterns comprising a plurality of graphical elements within the same process.

Without being bound by theory, it is believed that to create a plurality of controlled patterns using a plurality of rolls when the product pitch is greater than one half the largest allowed roll circumference, then the following may be observed:

First Roll (A) should have a circumference ($C_A$) = Product pitch (P)

Roll B: $C_B$= XP wherein 0<X< $C_{max}$/P where $C_{max}$ is the largest possible circumference that may be utilized in the process. If X is greater than 1, then one can determine the number of patterns by calculating how many times the multiple will repeat before it is a whole integer. That integer represents the number of patterns that may be created by that roll. For example, if X = 1.5, then

1.5 * 2 = 3. Hence, 3 is the number of potential patterns created by that roll before it repeats.

Similarly, if X = 1.7, then

1.7 * 10 = 17. Hence, 17 is the number of potential patterns created by that roll before it repeats.

If X is less than 1, then:

X should be chosen such that 1/X ≠ an Integer. If 1/X = an integer, then the additional roll will only create one product.

Provided that 1/X ≠ an Integer, then the number of patterns the additional roll may provide is equal to:

For example, if X= 0.8, then

0.8 * 5 = 4 patterns. Hence, 4 is the number of potential patterns created by that roll before it repeats.

wherein Integer = number of patterns before repeating

And (X-1) / (graph dimension/ product length) wherein X> equal to 1 to prevent cutting off during walking.

ROLL C: C=XP where 0<X<1

To create more than one pattern, 1/X cannot equal an integer.

Additionally, for either rolls B or C, the following must be true for the system to create multiple consistent patterns having a plurality of graphical elements:

$$\text{The Absolute of } ((X\text{-}1) / (\text{graphic dimension/ product length})) > 1$$

Utilizing the relationships above, it has been found that one can utilize two or more rolls in a flexographic or gravure printing process to create a plurality of consistent designs even when the product pitch is greater than one half of the largest possible circumference.

When utilizing three or more rolls wherein the second roll and the third roll each have a circumference that is a multiple of the first roll based on the equations above, and the circumference of the second roll is different than the circumference of the third roll, the number of patterns created is equal to the lowest possible integer that can be divided by the number of patterns each roll may provide to equal a whole number.

In further embodiments, the method is further characterized in that any number of additional rolls may be added provided that at least one roll has a circumference that is a multiple where X is greater than zero and less than the max circumference divided by the product pitch. For example, a backsheet may have a pattern that comprises of three distinct elements which are the same on all products and an additional element which varies in location on the backsheet. The additional element which varies in location will repeat after a fixed number of articles to create a consistent set of repeating patterns. The roll providing the additional element that varies in location has a circumference that is a multiple of, for example, between 1.1 and 2 of the other rolls. The changing elements created by the additional roll serves to distinguish the products such that they do not all have the same pattern. Additionally, the process may comprise more than one roll that has a circumference that is a multiple where X is greater than zero and less than the max circumference divided by the product pitch. Provided that the additional rolls do not have the same circumference, then the number of potential patterns may increase significantly, such as, for example, over 50 patterns. This allows for one to create a multiple of combinations depending on the circumference of each of the rolls that does not have a repeating fixed pattern.

In creating a multiple of patterns using a plurality of rolls, it is important that any rolls having more than one distinct pattern or element should have varying circumferences. Stated otherwise, if two rolls each have a multiple of the first roll and the multiple is the same, then due to web speed, the two multiple factor rolls would rotate at the same speed thereby creating a limited number of patterns. However, if the roll circumference of a second roll has a multiple factor of 1.5 and the roll circumference of the third roll has a multiple factor of 1.25, then the rolls will create 12 distinct combinations before repeating the patterns.

According to further embodiments, the number of items each having a different visually detectable feature provided before the pattern sequence of the visually detectable features starts to repeat itself is greater than the number of items imparted after one full turn of the roll. In other words, the method allows producing a large amount of items each having a different visually detectable feature using a two or more rolls. This is explained in greater detail below.

The roll may be an embossing roll, a printing roll, or an embossing and printing roll. The surface of the roll is provided with at least one visually detectable element. A visually detectable element may be a geometric or graphic pattern, a single geometric or graphic figure, a symbol or character, a coloured portion, a text message, an embossing pattern, an embossing figure or the like.

According to certain embodiments, the roll may include at least one first visually detectable element having a length equal to the length of the item, and at least one second visually detectable element having a length different from the length of the item, wherein said first and said second visually detectable elements are different. The first item imparted by such a roll will be provided with a first visually detectable feature including a first visually detectable pattern element corresponding to the first visually detectable element. The next (second) item will be provided with a second visually detectable feature including a second visually detectable pattern element corresponding to the second visually detectable element on the roll.

The roll may also include at least one first visually detectable element having a length different from the length of the item, and at least one second visually detectable element having a length different from the length of the item, wherein said first and said second visually detectable elements are different. Assuming that the lengths of the first and second visually detectable elements are shorter than the length of the item, the first item will be provided with a visually detectable feature including a portion of the first visually detectable pattern element and a portion of the second visually detectable pattern element. The second item will be provided with a visually detectable feature including a portion of the second visually detectable pattern element and a portion of the first visually detectable pattern element. The subsequent third item will be provided with a visually detectable feature including a portion of the first visually detectable pattern element and a portion of the second visually detectable pattern element, such that the visually detectable feature of the third item is different from the visually detectable features of preceding and following items.

In certain embodiments, the method may be used for imparting visually detectable features to items such as absorbent articles such as diapers, sanitary napkins, panty liners, incontinence protectors or the like. The items may also be wrapping sheets, such as single wraps or easy wraps. Further, the method may be used for imparting items such as release liners.

In cases when the method is used for imparting a visually detectable feature on an absorbent article, said visually detectable feature may be provided on at least one component of said absorbent article, such as a topsheet, a backsheet, a protective layer, an acquisition layer or an absorbent element.

The visually detectable feature may also be provided on at least two components of said absorbent article, for example, on the topsheet and the backsheet, such that said visually detectable feature on the first component is different from said visually detectable feature on the second component.

The items produced using methods according to methods described above will thus each have different visually detectable features. The items of a production series may then be placed in a package including a plurality of items wherein each item within said package includes a visually detectable feature, wherein said visually detectable feature includes at least one visually detectable pattern element, said visually detectable feature of each of said items is different from the visually detectable features of all other items within said package. The package can include a first item including a first visually detectable feature including at least a portion of a first visually detectable pattern element, and a second item includes a second visually detectable feature including at least a portion of said first visually detectable pattern element, wherein said first and second visually detectable features are different.

As explained above, by utilizing multiple rolls wherein at least one roll has a repeating pattern and wherein at least one roll has two or more patterns having visually distinct features, the package can include at least two items having different visually detectable features such that said visually detectable features include portions of the same visually detectable pattern element. Despite the fact that the visually detectable features of these items will include portions of the same visually detectable pattern element, these portions will be different.

A variety of visually detectable features within the same package has a certain emotional benefit for the user, since the user will be surprised by a new design at every change of item.

In certain embodiments, the package includes a plurality of items each having a different visually detectable feature. Each visually detectable feature in turn includes at least one visually detectable pattern element which may be a geometric or graphic pattern, a single geometric or graphic figure, a symbol or character, a colored portion, a text message, an embossed pattern, an embossed figure or the like.

The package may contain absorbent articles such as diapers, sanitary napkins, panty liners, incontinence protectors or the like.

The items within the package may also be wrapping sheets, such as single wraps or easy wraps, or release liners.

In cases when the package includes absorbent articles, said visually detectable feature may be provided on at least one component of said absorbent article, such as a topsheet, a backsheet, an acquisition layer or an absorbent element.

The visually detectable feature may also be provided on at least two components of said absorbent article, for example, on the topsheet and the backsheet, such that said visually detectable feature on the first component is different from said visually detectable feature on the second component.

The person skilled in the art would understand that the number of items each having a different visually detectable feature may be varied depending on the character of the item, the size of the package and so on. The number of items each having a different visually detectable feature in a package may, for example, be between 10 and 50.

Example 1: A Plurality of images are being created with 4 print cylinders of the following circumferences

Cylinder 1 = the pitch of the product (P)

$$\text{Cylinder } 2 = \text{Factor A} *P = 0.8 * P$$

$$\text{Cylinder } 3 = \text{Factor B} *P = 0.6 *P$$

$$\text{Cylinder } 4 = \text{Factor C} * P = 1.3* P$$

In this example the total number of images created can be calculated by expressing each factor as a fraction in its simplest form:

Factor A = 0.8 = 4/5 which means there will be 5 images printed from print cylinder 2 which will repeat every 4th product pitch set by Cylinder 1
Factor B = 0.6 = 3/5 which means there will be 5 images printed from print cylinder 3 which will repeat every 3rd product pitch set by Cylinder 1
Factor C = 1.3 = 13/10 which means there will be 13 images printed from print cylinder 4 which will repeat every 13th product pitch set by Cylinder 1.

Therefore the unique number of images created by this arrangement of cylinders is calculated by multiplying the number product pitches each print cylinder will repeat as set by cylinder 1. This will be the number of unique images that will be generated up to the point when all the individual images printed from each cylinder will be printed at the same original location creating a repeated image or artwork.

In this example then the number of unique artworks (N) = 4 *3*13 = 136 unique artworks.

Example 2: A Plurality of images are being created with 3 print cylinders of the following circumferences

Cylinder 1 = the pitch of the product (P)

$$\text{Cylinder } 2 = \text{Factor A} *P = 0.4*P$$

$$\text{Cylinder } 3 = \text{Factor B} *P = 1.5*P$$

Therefore
Factor A = 2/5 which means there will be 5 images printed from print cylinder 2 which will repeat every 2nd product pitch set by Cylinder 1
Factor B = 3/2 which means there will be 2 images printed from print cylinder 3 which will repeat every 3rd product pitch set by Cylinder 1
The unique number of artworks (N) = 2*3 = 6 unique artworks

Example 3: A Plurality of images are being created with 2 print cylinders of the following circumferences

Cylinder 1 = the pitch of the product (P)

$$\text{Cylinder } 2 = \text{Factor } A*P = 1.2*P$$

Therefore Factor A = 6/5 which means there will 5 images printed from print cylinder 2 which will repeat every $6^{th}$ product pitch set by Cylinder 1
The unique number of artwork N = 6 unique artworks

Example 4: A product with a pitch = 630mm is printed in a press where the maximum circumference of the print cylinder is 1 meter. The print pitch is > 0.5 * Cmax = 500mm, therefore a plurality of images will be achieved using the following cylinder arrangements:

Print cylinder 1 = 630mm circumference

$$\text{Print cylinder } 2 = \text{Factor } A * \text{Print pitch} = 1.5 * 630mm = 945mm$$

To ensure the whole graphics being printed are all fitting within the set product pitch (630mm), the following condition must be true for graphic length which for this example has been set to 100mm
Abs(Factor A -1) / (graphic length / print pitch) > 1
(1.5 - 1)/ (100mm/630mm) = 3.15 >1 therefore the condition is met
To calculate the unique number of artworks that could be printed
Factor A = 1.5 = 3/2 therefore 2 images will be printed from print cylinder 2 which will repeat every $3^{rd}$ product pitches set by Cylinder 1 resulting in 3 unique artworks.

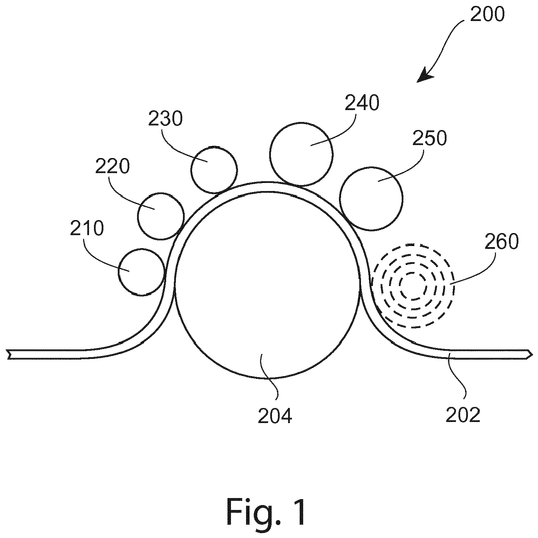

FIG. 1 illustrates a schematic of the process 200 wherein a first roll 210 and a second roll 220 are shown. The material web 202 comes in contact with both the first roll 210 and the second roll 220 while on a carrier drum 204. The first roll 210 comprises of a repeating pattern. The second roll 220 comprises of a different repeating pattern. As previously discussed, the rolls may be any one of embossing rolls, printing rolls (flexographic or otherwise), or any other type or roll by which a visually distinct element may be imprinted or added to a material web. As shown in FIG. 1, additional rolls 240, 250, and 260 may be used. Roll 260 is a representation of the possible roll diameters within the process. Any additional rolls may have the same pattern or a different pattern. For example, a fifth roll may have the same diameter, pitch, and pattern as the first roll. The roll may either be offset such that it prints the pattern adjacent to the original first roll pattern or aligned with the first roll such that it reinforces the first printed pattern.

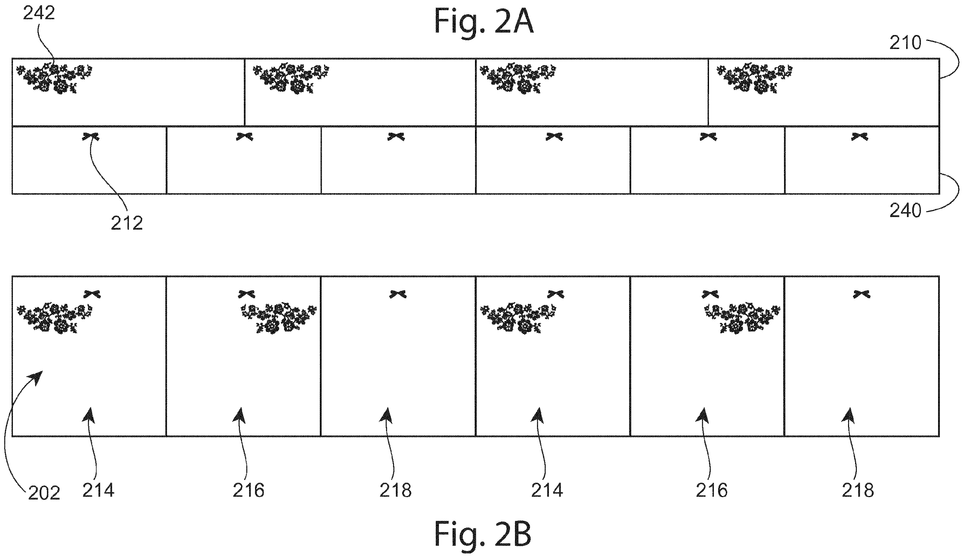

FIG. 2A-B shows a first roll 210 in a flat plan view, a second roll 240 in a flat plan view, and a web 202 in an outstretched flat view after contacting at least a first roll 210 and a second roll 240. As shown by FIG. 2A, the first roll 210 provides a consistent pattern exemplified by the printed bow 212. The second roll 240 is set to a multiple of the first roll and the product pitch such that three distinct patterns are created on the print pitch exemplified by the flowers 242. FIG. 2B shows the web 202 after contacting the first roll 210 and the second roll 240 thereby creating three specific repeating patterns. The combined roll effect creates a first pattern 214, a second pattern 216, and a third pattern 218 that are distinct and equal to the length of the product in the desired print direction (either machine direction or cross direction of the product). Specifically, a first pattern wherein the flowers are located on the left portion of the print pitch 214, pattern wherein the flowers are printed on the right side of the product pitch 216, and a pattern wherein the flowers are not printed 218. It has been found that by using a plurality of rolls wherein at least one roll has a plurality of patterns, one can increase the number of patterns without requiring additional separate manufacturing lines.

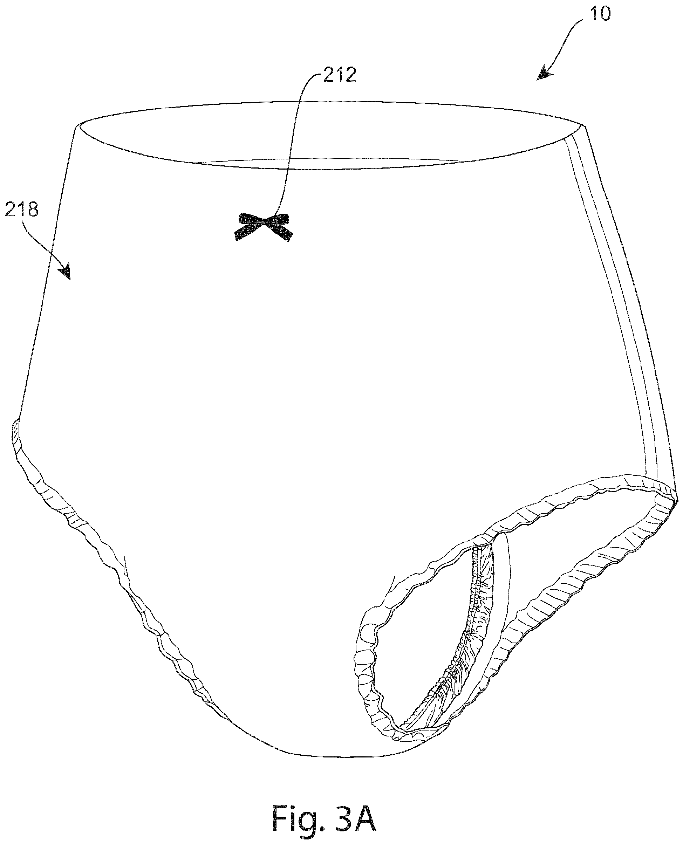

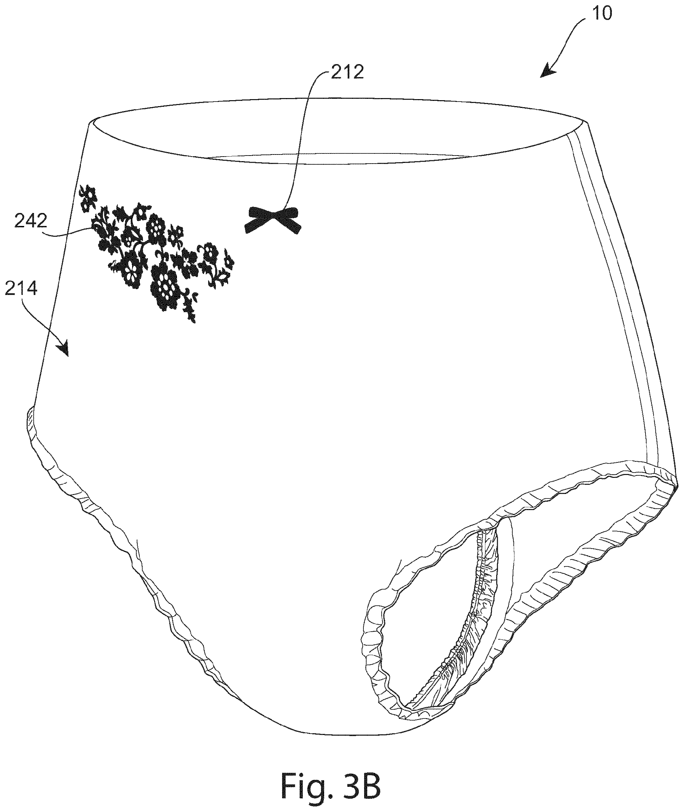

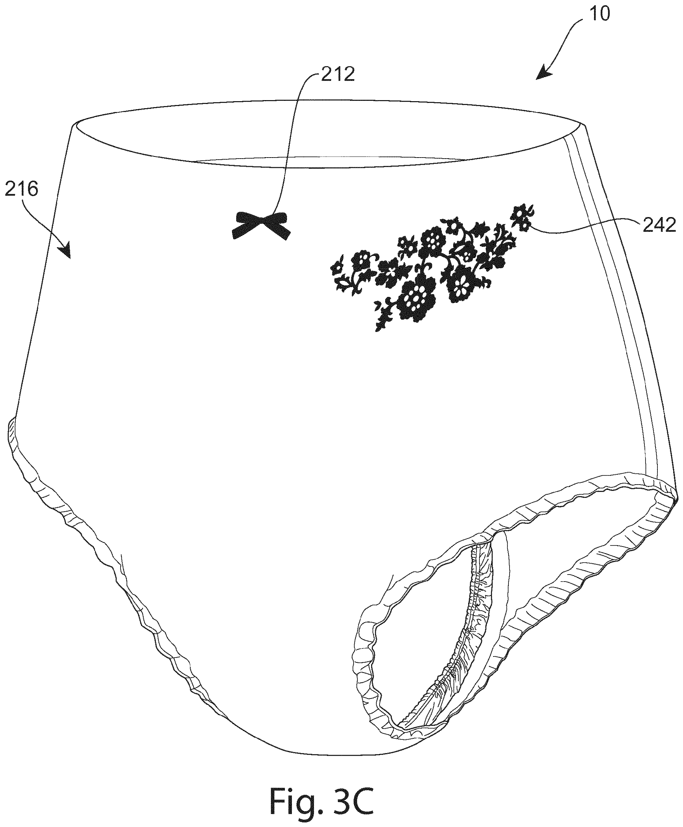

FIGS. 3A-C illustrates a perspective view of an absorbent article 10 having the three distinct patterns of FIG. 2. FIG. 3A represents the third pattern 218. FIG. 3B represents the first pattern 214. FIG. 3C represents the second pattern 216.

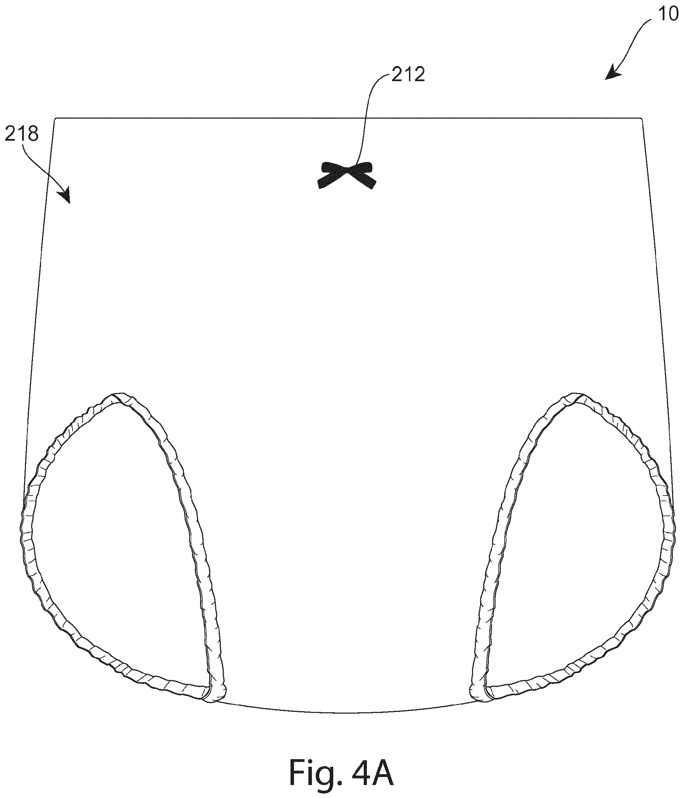

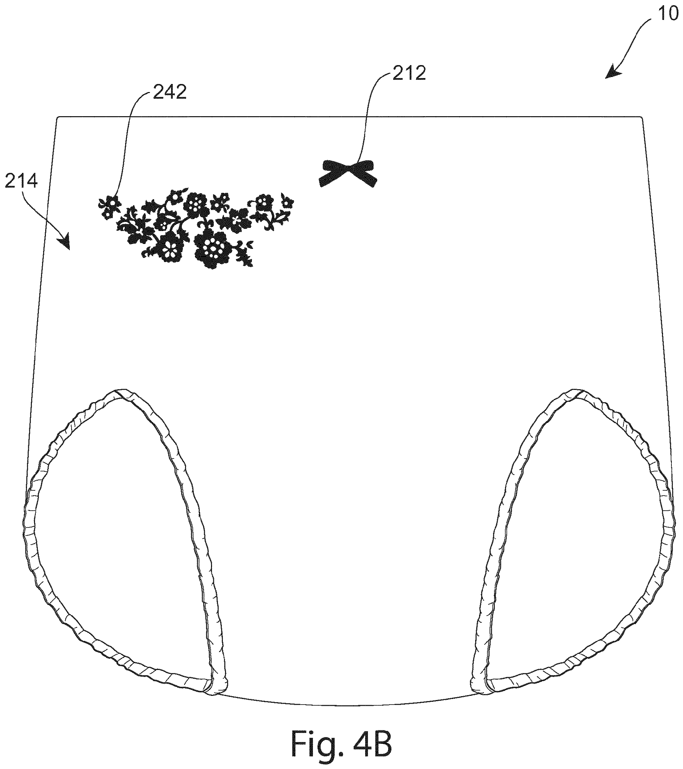

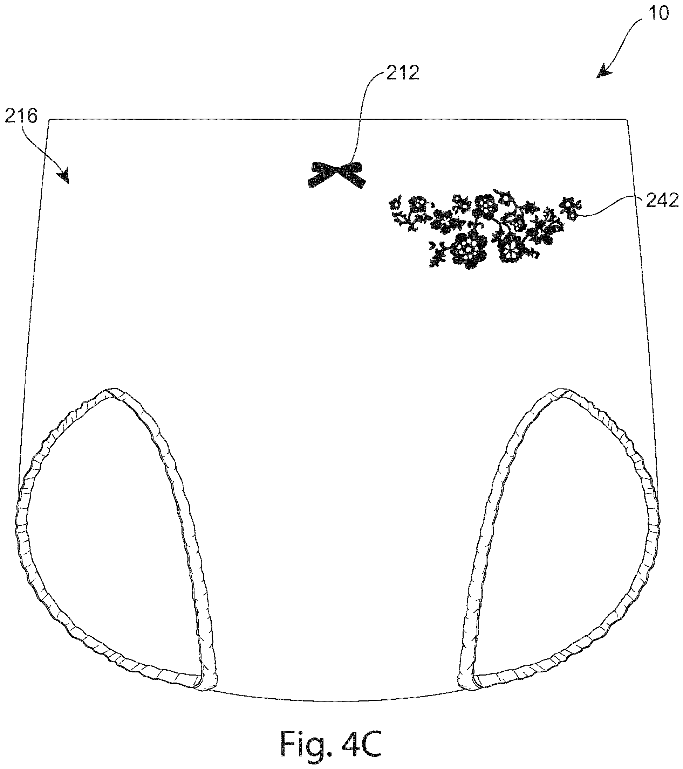

FIGS. 4A-C represent plan views of the absorbent article 10 of FIGS. 3A-C.

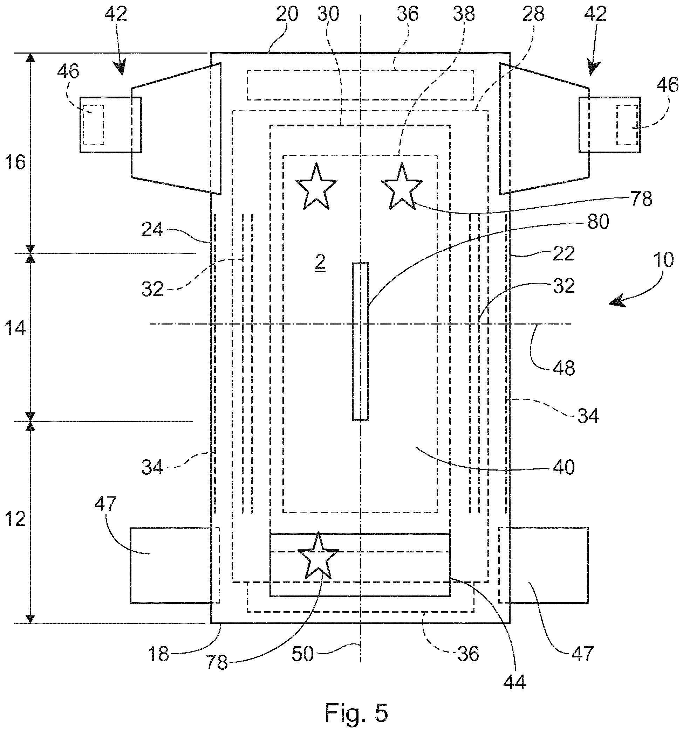

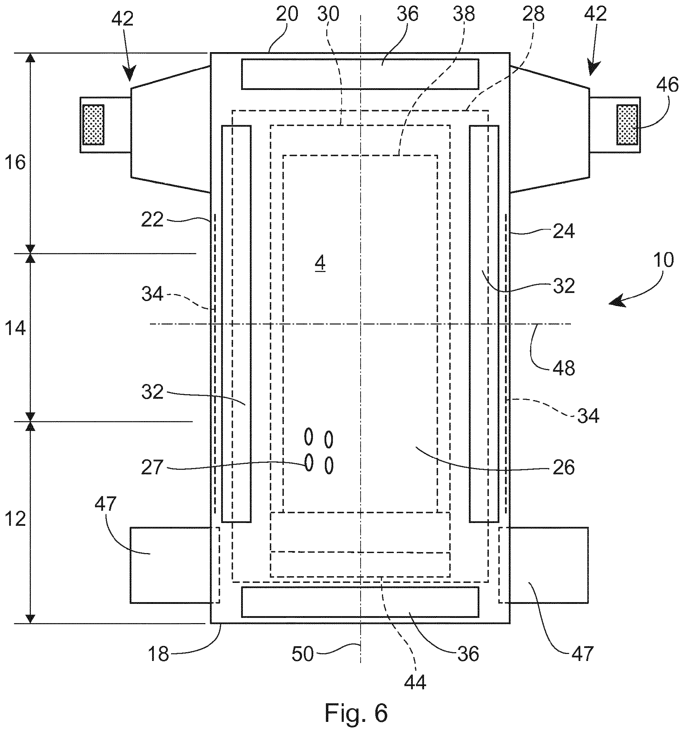

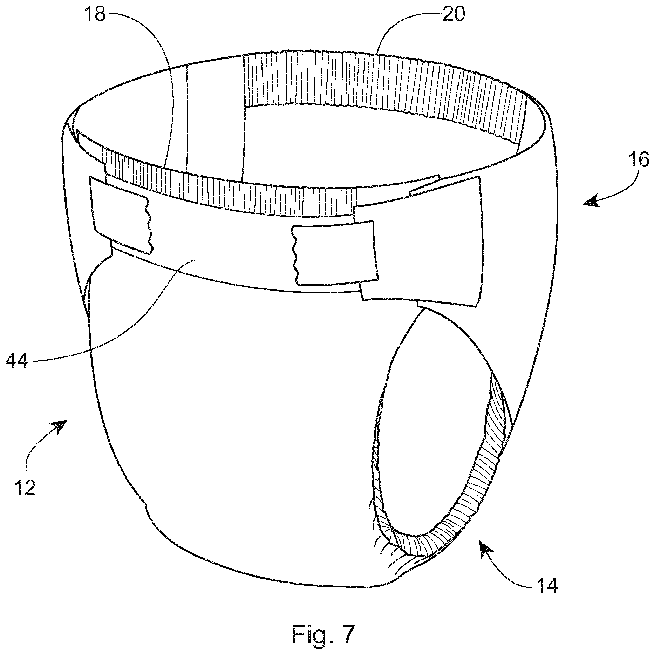

An example absorbent article according to the present disclosure, shown in the form of a taped diaper 10, is represented in Figs. 5-7. Fig. 5 is a plan view of the example diaper, garment-facing surface 2 facing the viewer in a flat laid-out state. Fig. 6 is a plan view of the example diaper of Fig. 5, wearer-facing surface 4 facing the viewer in a flat laid-out state. Fig. 7 is a front perspective view of the diaper of Figs. 5 and 6 in a fastened position. The diaper of Figs.

5-7 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The absorbent article 10 may comprise a front end edge 18, a back end edge 20 opposite to the front end edge 18, a first side edge 22, and a second side edge 24 opposite to the first side edge 22.

The absorbent article 10 may comprise a liquid permeable topsheet 26, a liquid impermeable backsheet 28, an absorbent core 30 positioned at least partially intermediate the topsheet 26 and the backsheet 28. The absorbent article 10 may also comprise one or more pairs of barrier leg cuffs 32, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40 may cover a garment-facing side of the backsheet 28. The absorbent article 10 may comprise back ears 42 in the back waist region 16 that may be attached to a landing zone area or landing zone material 44 in the front waist region 12. The back ears 42 may comprise fasteners 46 and may extend from the back waist region 16 of the absorbent article 10 and attach to the landing zone area or landing zone material 44 on a garment-facing portion of the front waist region 12 of the absorbent article 10, or *vice verso.* The absorbent article 10 may also have front ears 47 in the front waist region 12. The absorbent article 10 may have a lateral axis 48 and a longitudinal axis 50.

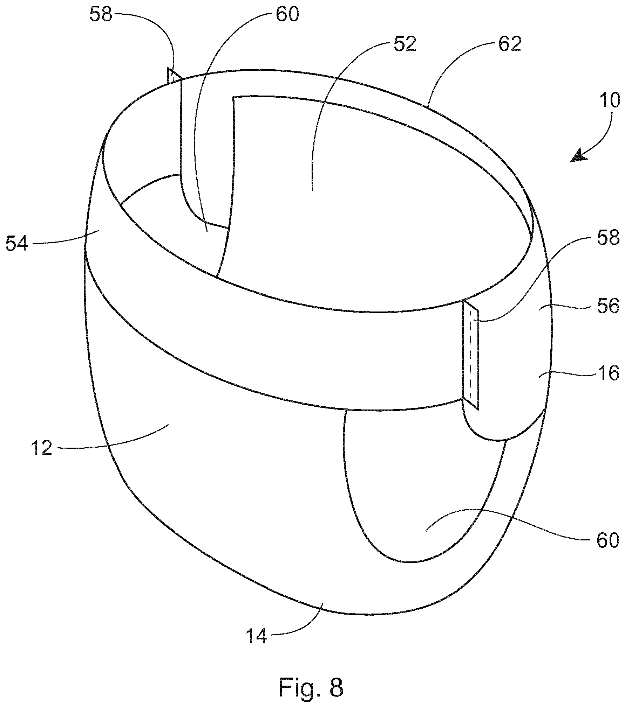

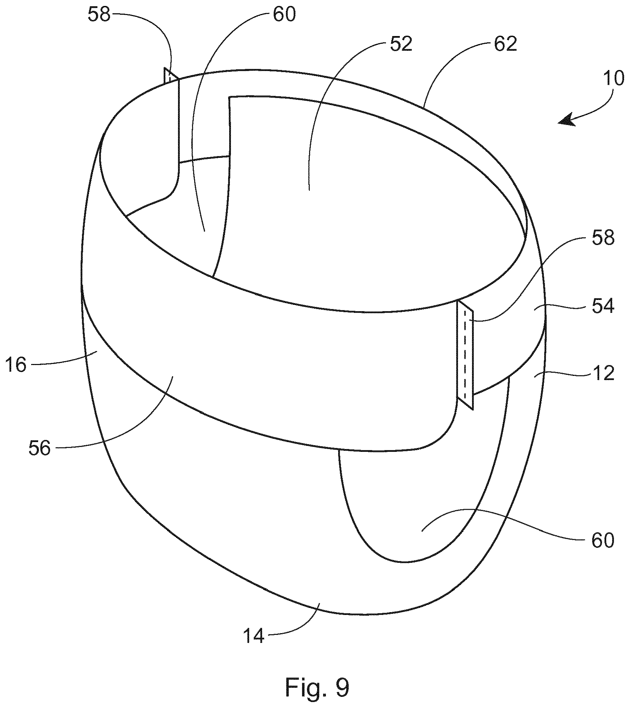

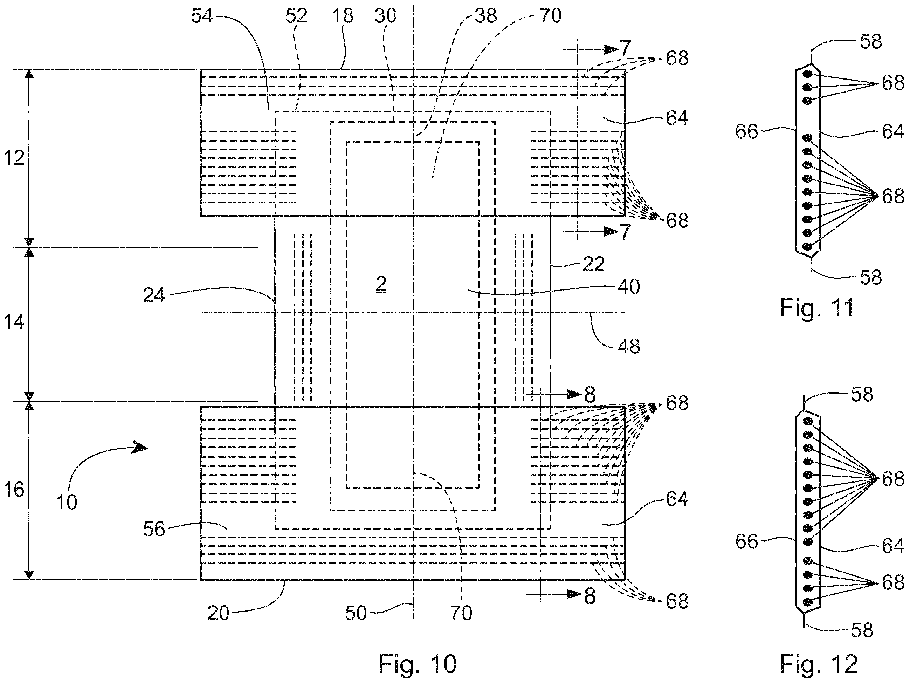

In other instances, the absorbent article may be in the form of a pant having permanent or refastenable side seams. Referring to Figs. 8-10, an example absorbent article in the form of a pant 10 is illustrated. Fig. 8 is a front perspective view of the pant 10. Fig. 9 is a rear perspective view of the pant 10. Fig. 10 is a plan view of the pant, laid flat, with the garment-facing surface facing the viewer. The pant 10 may have a front waist region 12, a crotch region 14, and a back waist region 16. The pant 10 may has a chassis 52 (sometimes referred to as a central chassis or central panel) comprising a topsheet 26, a backsheet 28, and an absorbent core 30 disposed intermediate the topsheet 26 and the backsheet 28, and an optional acquisition material 38, similar to that as described above with respect to Fig. 1-3. The pant 10 may comprise a front belt portion 54 in the front waist region 12 and a back belt portion 56 in the back waist region 16. The chassis 52 may be joined to a wearer-facing surface of the belt portions 54, 56 or to a garment-facing surface of the belt portions 54, 56. Side areas of the front belt portion 54 may be joined to side areas of the back belt portion 56 to form two seams 58. The seams 58 may be any suitable seams known to those of skill in the art, such as butt seams or overlap seams, for example. When the seams 58 are permanently formed or refastenably closed, the pant 10 has two leg openings 60 and a waist opening circumference 62. The seams 58 may be permanently joined using adhesives or bonds, for example, or may be refastenably closed using hook and loop fasteners, for example. The front belt portion 54 may comprise a first nonwoven material 64 and a second nonwoven material 66. A plurality of elastic elements 68 (e.g., elastic stands, elastic strips) may be positioned intermediate the first and second nonwoven materials 64, 66. In some instances, an elastic film may be used instead of, or in addition to, the elastic elements 68. The back belt portion 56 may comprise a first nonwoven material 64 and a second nonwoven material 66. A plurality of elastic elements 68 (e.g., elastic stands, elastic strips) may be positioned intermediate the first and second nonwoven materials 64, 66. In some instances, an elastic film may be used instead of, or in addition to, the elastic elements 68. The elastic elements 68 or film may be relaxed (including being cut) to reduce elastic strain over the core 30 or may alternatively run continuously across the core 30. Elements of Fig. 8-10 having the same reference number as described above with respect to Fig. 5-7 may be the same element (e.g., absorbent core 30). Fig. 11 is a cross-sectional view of the front belt portion 54 taken about line 7---7 of Fig. 10. Fig. 12 is a cross-sectional view of the back belt portion 56 taken about line 8-8 of Fig. 10. The elastics elements 68 may have uniform or variable spacing therebetween in any of the belt portions. The elastic elements may also be pre-trained the same amount or different amounts. The first and/or second belt portions 54 and 56 may have one or more elastic element free zones 70 where the chassis 52 overlaps the belt portions 54, 56. In other instances, at least some of the elastic elements 68 may extend across the chassis 52.

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

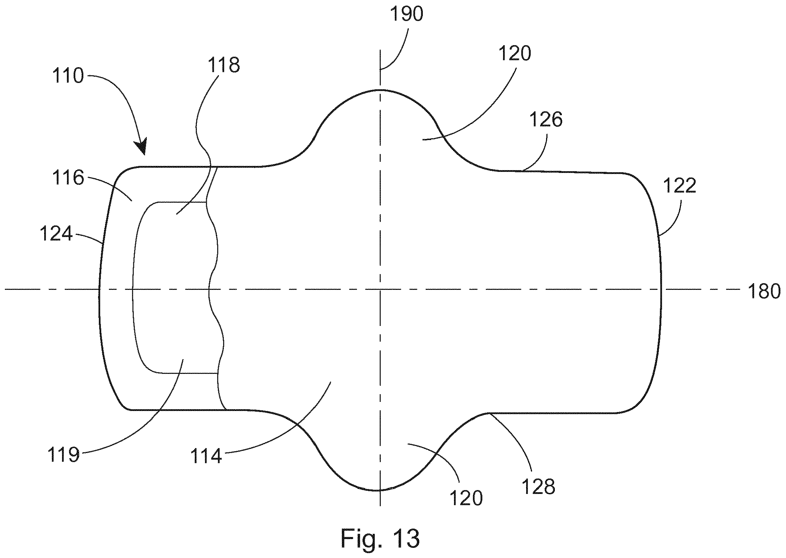

Referring to Fig. 13, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

Although the present invention has been described with reference to various embodiments, those skilled in the art will recognize that changes may be made without departing from the scope of the invention. It is intended that the detailed description be regarded as illustrative and that the appended claims including all the equivalents are intended to define the scope of the invention.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A method of continuously imparting different visually detectable features to a plurality of articles, said method comprising:

Providing a printing unit comprising of two or more printing rolls each having a visually detectable pattern, wherein the printing process may fit rolls that are less than or equal to a maximum circumference;
providing a web comprising a print pitch;
providing a first roll wherein the roll circumference is equal to the product pitch;
providing a second roll **characterized in that** the roll circumference is equal to either $0<X<1$ where X is chosen such that $1/X \neq$ an Integer or $1<X< C_{max}/P$,
wherein X is a multiple of the first roll circumference, $C_{max}$ is the largest circumference allowable by the printing unit, and P is the product pitch; and imparting said visually detectable patterns to the web by means of the one or more rolls,
wherein the method is **characterized in that** each of the first roll and the second roll comprises a visually detectable pattern defining a print length, wherein the print length is **characterized in that** the absolute of (X-1) / (print length/ product pitch)) is greater than 1.

**2.** The method according to claim 1, **characterized in that** each of the first roll and the second roll comprises a visually detectable pattern defining a print length, wherein the print length is **characterized in that** the absolute of (X-1) / (print length/ product pitch)) is greater than 1.

**3.** The method according to claim 1 or 2, wherein the method further comprises one or more additional rolls wherein each of the one or more additional rolls are **characterized in that** each roll circumference is equal to either $0<X<1$ where X is chosen such that $1/X \neq$ an Integer or $1 <X< C_{max}/P$, wherein X is a multiple of the first roll circumference, $C_{max}$ is the largest circumference allowable by the printing unit, and P is the product pitch.

**4.** The method according to claim 3, wherein at least one of the one or more rolls is an embossing roll.

**5.** The method according to claim 4, wherein at least one of the one or more rolls is a print roll.

**6.** The method according to any of claims 1-5, wherein each roll prints a visually detectable feature that is a printed and/or embossed pattern, a printed and/or embossed image, a colour or a combination thereof.

**7.** The method according to any of claims 1-6, **characterized in that** in said web comprises a first product pitch having a first pattern comprising a first visually detectable feature and a second visually detectable feature and a second product pitch having a second pattern comprising the first visually detectable feature and the second visually detectable feature.

**8.** The method according to any of claims 1-7, wherein the web is a portion of an absorbent article such as a diaper, a sanitary napkin, a panty liner, an incontinence protector or the like.

**9.** The method according to any of claims 1-8, wherein the web is a wrapping sheet, such as a single wrap or an easy wrap.

**10.** The method according to any of claims 1-9, wherein the web is a release liner.

**11.** The method according to claim 7, **characterized in that** said visually detectable feature is provided on at least one component of said absorbent article, such as a topsheet, a backsheet, an acquisition layer or an absorbent element.

**12.** The method according to claim 7, **characterized in that** said visually detectable feature is provided on at least two components of said absorbent article, said visually detectable feature on the first component being different from said visually detectable feature on the second component.

**13.** The method according to any of the preceding claims 1-12, wherein the print pitch is greater than one half the maximum circumference.

Fig. 1

Fig. 2A

242

210

212

240

Fig. 2B

202  214        216        218        214        216        218

EP 3 326 598 A1

Fig. 3A

Fig. 3B

Fig. 3C

Fig. 4A

10

212

242

214

Fig. 4B

10

212

216

242

Fig. 4C

Fig. 5

Fig. 6

Fig. 7

Fig. 8

Fig. 9

Fig. 10

Fig. 11

Fig. 12

Fig. 13

Europäisches
Patentamt

European
Patent Office

Office européen
des brevets

# EUROPEAN SEARCH REPORT

Application Number

EP 17 20 3266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/046591 A1 (WARNER ALRICK VINCENT [US]) 24 February 2011 (2011-02-24) * paragraphs [0049] - [0084]; claim 1; figures * | 1-13 | INV. A61F13/514 A61F13/84 A61F13/15 B41F5/18 B41F5/24 |
| X | WO 2006/007031 A1 (KIMBERLY CLARK CO [US]; LARSON TODD C [US]; MLEZIVA MARK M [US]; HOEHN) 19 January 2006 (2006-01-19) * paragraphs [0007] - [9;46]; claims; figures * | 1-13 | |
| X | US 2010/331799 A1 (SCHNEIDER UWE [US]) 30 December 2010 (2010-12-30) * paragraphs [0029] - [0039]; claims; figures * | 1-13 | |

TECHNICAL FIELDS
SEARCHED (IPC)

A61F
B41F

The present search report has been drawn up for all claims

| Place of search | Date of completion of the search | Examiner |
|---|---|---|
| Munich | 22 January 2018 | Boccignone, Magda |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 3266

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2018

| Patent document cited in search report | | Publication date | Patent family member(s) | | Publication date |
|---|---|---|---|---|---|
| US 2011046591 | A1 | 24-02-2011 | BR 112012002878 | A2 | 22-03-2016 |
| | | | CA 2771611 | A1 | 24-02-2011 |
| | | | CN 102481214 | A | 30-05-2012 |
| | | | EP 2340003 | A1 | 06-07-2011 |
| | | | JP 2012506331 | A | 15-03-2012 |
| | | | US 2011046591 | A1 | 24-02-2011 |
| | | | US 2016051422 | A1 | 25-02-2016 |
| | | | WO 2011022537 | A1 | 24-02-2011 |
| WO 2006007031 | A1 | 19-01-2006 | US 2006092431 | A1 | 04-05-2006 |
| | | | WO 2006007031 | A1 | 19-01-2006 |
| US 2010331799 | A1 | 30-12-2010 | US 2010331799 | A1 | 30-12-2010 |
| | | | WO 2010151398 | A1 | 29-12-2010 |

EPO FORM P0459

For more details about this annex : see Official Journal of the European Patent Office, No. 12/82